# FASCICULE DE BREVET EUROPEEN

(11) **EP 2 088 935 B1**
(45) Date de publication et mention de la délivrance du brevet: **21.03.2018**
(21) Numéro de dépôt: 07858500.7
(22) Date de dépôt: 03.10.2007
(51) Int. Cl.: A61B 17/04

(54) **IMPLANT D'ANCRAGE ET DE FIXATION ET ENSEMBLE CHIRURGICAL POUR SON IMPLANTATION**
VERANKERUNGS- UND FIXIERUNGSIMPLANTAT UND CHIRURGISCHE ANORDNUNG ZU SEINER IMPLANTATION
ANCHORING AND FIXING IMPLANT AND SURGICAL ASSEMBLY FOR ITS IMPLANTATION

(30) Priorité: 04.10.2006 FR 0608703
(43) Date de publication de la demande: 19.08.2009
(73) Titulaire: Textile Hi-Tec (T.H.T.), 34220 Verreries de Moussans (FR)
(72) Inventeur: HOUARD, William, 81270 Labastide-Rouairoux (FR); BERRET, Philippe, 34570 St Paulet Valmalle (FR); FONSECA, Fabrice, 13780 Cuges les Pins (FR); BAUTRANT, Eric, 13540 Purycard (FR)
(74) Mandataire: Balesta, Pierre
(86) Numéro de dépôt international: PCT/FR2007/052067
(87) Numéro de publication internationale: WO 2008/040914

(56) Documents cités:
- US-A- 5 030 224
- US-A- 5 203 787
- US-A- 5 573 543
- US-B1- 6 174 323

## Description

La présente invention concerne le domaine des implants chirurgicaux. Elle concerne plus particulièrement un implant permettant de réaliser l'ancrage et la fixation, voire la mise en tension, d'autres implants, notamment bandelettes ou plaques prothétiques dans des tissus, cette fixation et cette mise en tension étant réalisées avec au moins un fil de suture.

L'utilisation d'implants d'ancrage et de fixation est une pratique tout à fait courante lorsque le chirurgien doit installer à demeure une plaque prothétique ou une bandelette, sans risque de migration de celle-ci. Un tel implant doit remplir deux fonctions. Il doit tout d'abord permettre le passage, le coulissement et le blocage du fil de suture. Cette première fonction est généralement remplie par la présence dans l'implant d'une ouverture dans laquelle peut être enfilée le fil de suture, sans risque qu'il n'en sorte lors des manipulations du praticien. La seconde fonction réside dans la capacité d'ancrage, c'est-à-dire de blocage en position dans les tissus. Un tel implant d'ancrage et de fixation doit également être de taille réduite de manière à ce que sa présence ne constitue pas une gêne en tant que tel. On a déjà proposé sur le marché un grand nombre de modèles différents d'implants d'ancrage et de fixation dont certains sont constitués d'un fil continu avec des portions rectilignes servant à remplir la fonction d'ancrage et des portions recourbées servant notamment à former, par repliement, une ouverture servant à la fonction de fixation du fil.

Dans le document WO 2005/041784 A1, l'implant qui est illustré à la figure 1 comporte une portion recourbée faisant sensiblement les trois quarts d'un cercle prolongé par deux portions rectilignes se croisant à angle droit et se terminant par des extrémités pointues.

Dans le document US 5 203 787, l'implant comporte une portion centrale recourbée formant un cercle complet, et deux portions rectilignes, opposées l'une à l'autre et terminées par des extrémités effilées.

Dans ces deux modes de réalisation, l'ouverture pour le passage du fil de suture est constituée par la portion recourbée en arc de cercle, le praticien n'étant pas strictement obligé d'enfiler le fil de suture à travers l'ouverture comme dans le chas d'une aiguille mais pouvant l'introduire en faisant glisser une partie de ce fil entre les deux portions rectilignes jusqu'à pénétration dans l'ouverture. Il y a cependant un risque que, pendant les manipulations, lors de l'opération, le fil se désolidarise de l'implant en sortant de la portion recourbée par le même chemin qu'il avait emprunté lors de son introduction. Pour éviter ce risque, dans le document WO 2005/041784, il est prévu un système de blocage des deux portions rectilignes dans leur état superposé, mais cette solution rend plus complexe la fabrication de l'implant.

L'implant décrit dans le document US 5 501 683 comporte trois portions coudées faisant chacune un arc de cercle de 180°, à savoir une portion médiane et deux portions latérales lui faisant face mais légèrement décalée. Ainsi, les portions rectilignes qui sont dans le prolongement des dites portions coudées sont toutes parallèles entre elles. Les portions rectilignes de la portion médiane rejoignent les portions rectilignes internes des deux portions latérales en formant des plis à 45°, qui s'imbriquent l'un dans l'autre. Ce sont les deux portions rectilignes latérales externes qui servent à l'ancrage de l'implant dans le tissu. Pour ce faire, il est nécessaire de mettre en oeuvre un ancillaire qui d'une part soit capable d'introduire l'implant dans sa configuration au repos, avec les deux portions rectilignes latérales externes parallèles l'une à l'autre et qui d'autre part et surtout soit capable d'écarter les deux dites portions latérales externes de manière à les éloigner l'une de l'autre, réalisant ainsi la pénétration de leurs extrémités dans les tissus, de part et d'autre du trou d'implantation préalablement foré. Quant au fil de suture, il n'est pas introduit dans l'ouverture formée par la portion recourbée médiane mais dans la partie en U délimitée entre les deux portions rectilignes externes latérales. L'ouverture formée par la portion recourbée médiane sert, dans ce document US.5.501.683, à la préhension de l'implant par une pièce mobile de l'ancillaire, permettant la translation vers l'arrière dudit implant tandis que les portions rectilignes latérales externes viennent en appui contre une surface oblique, réalisant ainsi l'écartement souhaité.

Dans le document US.6,174,323, l'implant comporte une portion centrale recourbée en demi-cercle. Un moyen de blocage, par exemple une agrafe, est nécessaire pour assurer le blocage des deux brins du fil de suture en amont de l'implant (référence 50 sur la figure 16) et donc pour éviter que le fil de suture ne se désolidarise de l'implant.

Le but visé par la présente invention est de proposer un implant d'ancrage et de traction qui soit de réalisation et de mise en place simplifiées par rapport à celui décrit dans le document US.5.501.683 et qui pallie les inconvénients des implants proposés dans les documents WO.2005/041784, US.5.203.787 et US.6,174,323.

Il s'agit de manière connue d'un implant d'ancrage et de fixation qui est constitué d'un fil continu avec des portions rectilignes et des portions recourbées.

De manière caractéristique, cet implant comporte :
a) une portion centrale recourbée formant un enroulement jointif d'au moins sensiblement 1,5 spire,
b) de chaque côté et en prolongement de ladite portion centrale, un jeu de deux portions rectilignes, à savoir une portion intermédiaire et une portion d'extrémité, qui sont reliées l'une à l'autre par une portion coudée selon un angle faisant de l'ordre de 30 à 90°.

De plus les portions rectilignes d'extrémité sont orientées de manière divergente en sorte de faire entre elles un angle de l'ordre de 60 à 180°.

Dans une variante de réalisation, l'enroulement jointif de la portion centrale recourbée forme sensiblement une spire et demie. Cette disposition particulière permet une simplification de la fabrication et une réduction de l'encombrement de l'implant. Elle peut également permettre l'introduction du fil de suture en le faisant coulisser entre les deux portions rectilignes et glissement le long de la spire et demie jusqu'à pénétration dans l'orifice central de ladite spire. Le fait qu'il s'agisse d'une spire et demie et non plus d'une portion recourbée en arc de cercle faisant au plus 180° permet d'éviter la sortie involontaire du fil de suture de cette ouverture.

De préférence le fil continu est dans un matériau à mémoire de forme ce qui permet aux portions recourbées de faire office d'articulations, en sorte que, dans une position d'introduction, les deux portions rectilignes d'extrémité peuvent être contraintes l'une vers l'autre de manière à former entre elles un angle de l'ordre de 10 à 30° et, dans une position d'implantation, retrouver la disposition angulaire initiale de l'ordre de 60 à 180°.

Selon une variante de réalisation, la longueur des portions rectilignes d'extrémité est inférieure à la longueur des portions rectilignes intermédiaires. Comme cela pourra être mieux compris à la lecture de la description qui sera faite ci-après d'un exemple d'ancillaire d'implantation, cette disposition permet de réaliser l'ancrage de l'implant dans le tissu grâce au déploiement des deux portions d'extrémité, tout en maintenant partiellement l'implant dans une direction longitudinale à l'intérieur de l'ancillaire, avant sa totale libération.

Selon une variante de réalisation, les portions rectilignes intermédiaires font entre elles un angle de l'ordre de 10 à 30°. De préférence, dans ce cas, les centres des trois portions recourbées sont dans le même plan. C'est cette configuration qui est préférée qui sera décrite ci-après.

La présente invention a également pour objet un ensemble chirurgical qui comporte :
- un implant d'ancrage et de fixation présentant tout ou partie des caractéristiques précitées,
- un fil de fixation destiné à être enfilé dans la portion centrale recourbée de l'implant,
- un ancillaire de pose se présentant sous la forme d'un tube creux comportant une cavité ouverte à son extrémité distale, apte à servir de logement à l'implant dans une position d'introduction dans laquelle il a sa portion centrale recourbée dans le fond de la cavité, et ses portions rectilignes d'extrémité qui sont contraintes l'une vers l'autre en appui sur la paroi latérale de ladite cavité.

Pour l'évacuation de l'implant hors de la cavité , il suffit à l'opérateur de tendre le fil de fixation pour faire remonter la portion centrale recourbée et faire sortir de la cavité les portions rectilignes d'extrémité.

De préférence dans l'ensemble chirurgical pour la mise en place d'un dispositif chirurgical, notamment bandelette ou plaque prothétique, avec deux implants d'ancrage et de fixation et deux fils de fixation enfilés chacun dans la portion centrale recourbée d'un implant, au moins un fil de fixation a un premier brin non fixé audit dispositif qui forme un noeud coulissant autour du second brin fixé audit dispositif, apte à réaliser la mise en tension du dispositif entre les deux implants.

La présente invention sera mieux comprise à la lecture de la description qui va être faite d'un exemple préféré de réalisation d'un implant d'ancrage et de fixation, illustré par le dessin annexé dans lequel :
La figure 1 est une représentation schématique de côté dudit implant,
Les figures 2, 3 et 4 sont des représentations schématiques en perspective de l'implant de la figure 1 et de son ancillaire de pose, en position d'introduction (figure 2), en position intermédiaire d'évacuation (figure 3) et en position d'implantation (figure 4),
Les figures 5 et 6 sont des représentations schématiques de deux exemples de mise en place d'une bandelette à l'aide de deux implants, et
La figure 7 est une représentation d'un noeud coulissant sur un fil de fixation selon la figure 5.

L'implant 1 a pour fonction d'assurer l'ancrage dans des tissus, notamment osseux, d'un autre implant tel que bandelette ou plaque prothétique, à l'aide d'un fil de liaison, ou fil de suture. L'implant 1 est formé à partir d'un fil rigide continu de section circulaire, par exemple faisant environ 0,5 mm de diamètre, ou polygonale. Ce fil est dans un matériau stable et bio inerte, par exemple en alliage métallique notamment un alliage de titane, acier inoxydable ou alliage nickel titane. Il peut également être dans un matériau résorbable, par exemple à partir de polymère d'origine naturelle ou synthétique dont le polylactide, polyglycolide, poly-ε-caprolactone, polyhydroxy butyrate, polyhydroxy valérate, polycarbonates et assimilés, cellulose, polysaccharides, amidon, leurs homopolymères, leurs copolymères et leurs dérivés.

Dans le cas de matériaux résorbables, il est souhaitable que les propriétés mécaniques, en terme de force de rupture de l'implant, évoluent dans les conditions suivantes, à savoir à l'issue d'un délai de six mois après l'implantation perte de l'ordre de 50% des propriétés initiales et au bout d'un an perte de 100 % des propriétés initiales.

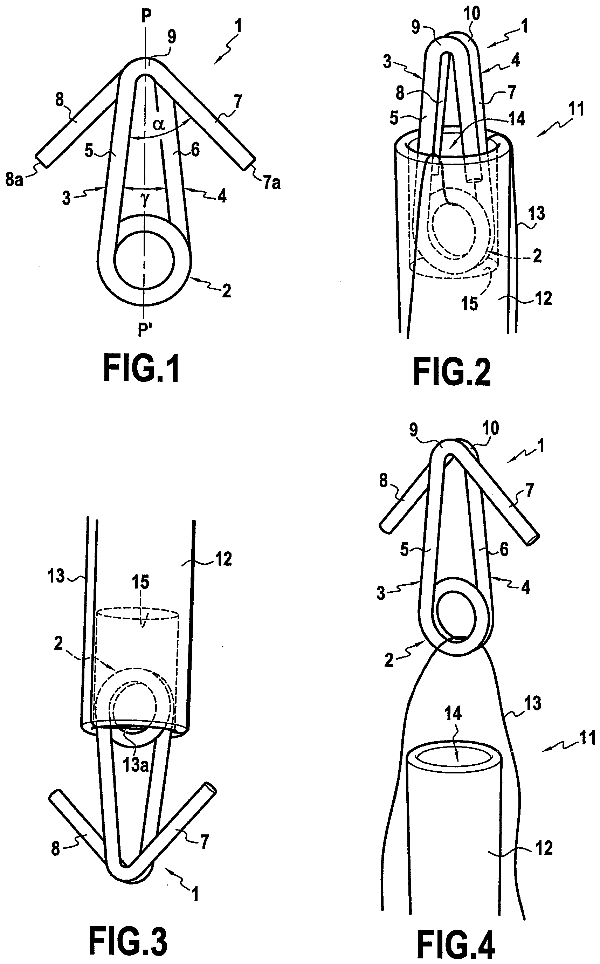

Le fil rigide continu en question est conformé sur un matériel automatique spécialisé pour prendre la forme telle qu'illustrée à la figure 1 qui correspond à un enchaînement continu de segments rectilignes et de portions recourbées.

Plus précisément l'implant 1 comporte :
a) une portion centrale 2 recourbée en sorte de former un enroulement jointif constitué d'au moins sensiblement 1,5 spire. Le nombre de spires n'est pas visible sur la figure 1, en vue de côté, mais il apparaît clairement sur les figures 2 à 4, sachant que dans l'exemple illustré, la portion centrale recourbée 2 ne comportant que sensiblement une spire et demie ;
b) de chaque côté et en prolongement de ladite portion centrale 2 un jeu de deux portions rectilignes 3,4, à savoir un segment intermédiaire 5, 6 et un segment d'extrémité 7,8, qui , dans chaque jeu sont reliés l'un à l'autre par une portion coudée 9, 10, ledit coude faisant un angle α de l'ordre de 30 à 90°.

De plus les deux segments d'extrémité 7, 8 sont orientés de manière divergente en sorte de faire entre eux un angle β de l'ordre de 60 à 180°.

Si la courbure donnée à la portion centrale 2 faisait strictement 1,5 spire, les deux segments intermédiaires 5, 6 seraient strictement parallèles. Tel n'est pas le cas dans l'exemple illustré pour lequel cet enroulement est légèrement supérieur à une spire et demie de telle manière que les deux segments intermédiaires 5, 6 forment entre eux un angle γ de l'ordre de 10 à 30°. De plus les centres de courbure des trois portions recourbées 2, 9, 10 sont dans le même plan PP' ce qui permet d'avoir, pour l'implant 1, une structure particulièrement compacte et un comportement parfaitement symétrique.

La présence des trois centres de courbure dans le même plan pourrait être obtenue avec une portion centrale 2 faisant strictement 1,5 spire, mais dans ce cas, il serait nécessaire d'augmenter le rayon de courbure des portions coudées 9, 10.

La longueur L0 des segments d'extrémité 7, 8 est inférieure à la longueur L1 des segments intermédiaires 5, 6. Cette disposition n'est pas strictement obligatoire mais permet d'éviter que lesdits segments d'extrémité 7, 8 viennent, à l'état replié d'introduction, s'appliquer sur la portion centrale recourbée 2, ce qui pourrait augmenter l'encombrement de l'implant et nécessiter donc de forer un trou d'implantation dans le tissu d'un diamètre plus important.

Les bouts 7a, 8a des segments d'extrémité 7, 8 pourraient éventuellement être effilés mais il est jugé préférable qu'au contraire ils ne soient pas traumatiques pour les tissus. De ce fait ils sont exempts d'arêtes vives ayant leurs bords légèrement arrondis.

Dans un exemple précis de réalisation, donné à titre non exhaustif, l'implant 1 est réalisé à partir d'un fil rigide faisant un diamètre de l'ordre de 0,5 mm. Sa longueur totale, selon le plan PP' est de l'ordre de 8 mm. Le diamètre externe de la partie centrale recourbée est de l'ordre de 3 mm. La longueur L0 des deux segments d'extrémité 7, 8 est de l'ordre de 4 mm. L'angle α est de l'ordre de 50°, l'angle β est de l'ordre de 90° et l'angle γ est de l'ordre de 15°.

En position au repos de l'implant 1, la distance séparant les bords extérieurs des bouts 7a, 8a des segments d'extrémité 7, 8 est de l'ordre de 7 mm.

Pour la mise en place de l'implant 1 il est prévu un ancillaire 12 de conception particulièrement simplifiée puisqu'il comprend un simple tube cylindrique présentant une cavité distale 14.

De préférence l'ensemble chirurgical 11 qui est proposé à l'opérateur est à usage unique, dans un emballage stérilisé, cet ensemble comportant l'implant d'ancrage et de fixation 1, en position repliée, logé à l'intérieur de l'ancillaire 12, avec le fil de fixation 13 préalablement passé à l'intérieur de la portion centrale recourbée 2 de l'implant 1.

Dans cet ensemble chirurgical 11, l'implant 1 est logé dans la cavité distale ouverte 14 au fond 15 de laquelle est placée la portion centrale recourbée 2.

Le diamètre de cette cavité 14 est légèrement supérieur au diamètre externe de la portion centrale recourbée 2.

Pour le placement de l'implant 1 dans la cavité 14, on procède à une action de compression des deux segments d'extrémité 7, 8 de manière à les rapprocher l'un de l'autre, en réduisant l'angle β à une valeur qui est proche de celle de l'angle γ de manière à ce que l'écartement des bords extrêmes 7a, 8a desdits segments 7, 8 soit équivalent au diamètre externe de la partie centrale 2. On peut ainsi introduire dans la cavité 14 l'implant 1, y compris au moins partiellement les segments d'extrémité 7, 8.

Sur la figure 2 on a représenté l'implant 1 logé partiellement à l'intérieur de la cavité 14, avec les deux segments d'extrémité 7, 8 repliés, les bouts 7a, 8a des deux dits segments venant en contact avec la paroi intérieure de la cavité 14. On comprend que ce sont les portions coudées 9, 10 qui jouent le rôle d'articulations pour réaliser la réduction angulaire entre les segments intermédiaires 5, 6 et les segments d'extrémité 7,8.

Une fois l'implant 1 introduit à l'intérieur de l'ancillaire, les segments d'extrémité 7, 8 tendent naturellement à revenir vers leur position angulaire initiale et en sont empêchés par la paroi interne de la cavité. La force d'application qui est donc exercée par ces segments assure le maintien de l'attache 1 à l'intérieur de l'ancillaire 12, quel que soit le positionnement de celui-ci.

Pour l'implantation de l'implant 1, le praticien fore tout d'abord un trou dans les tissus. Ce trou est généralement du diamètre externe de la partie distale de l'ancillaire 12 de manière à pouvoir y faire pénétrer ladite partie distale incluant l'implant 1. Il exerce une traction sur les deux brins du fil de fixation, dépassant de l'ancillaire 12, ce qui met en tension la portion 13a du fil qui était à l'intérieur de la cavité 14, comme illustrée à la figure 3, fait remonter la portion centrale recourbée 2 et sortir de la cavité 14 les portions d'extrémité 7,8. Sur la figure 3 est représenté le moment où l'implant 1 reste solidaire de l'ancillaire 12 par sa portion centrale recourbée mais où les deux segments d'extrémité 7, 8, libérés de la contrainte de la paroi interne de la cavité 14 sont revenus vers leur position angulaire initiale d'angle α et ont pénétré dans les tissus formant en quelque sorte deux crochets de harpon.

La figure 4 représente l'étape finale où l'implant 1 étant ancré dans les tissus, le praticien retire l'ancillaire 12 et peut utiliser le fil de fixation 13 pour réaliser la fixation définitive de l'autre implant en se servant de cet implant d'ancrage 1.

L'implant 1 et son ancillaire de pose 12 sont destinés à la chirurgie, que ce soit la chirurgie orthopédique, digestive , urologique ou encore gynécologique et que ce soit par voie artroscopique ou conventionnelle.

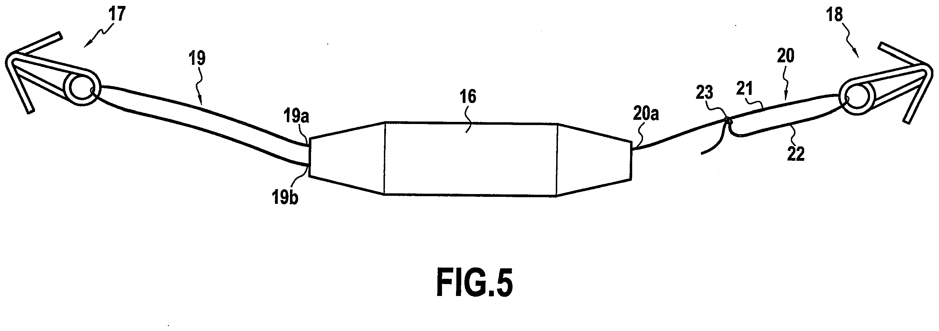

La figure 5 illustre très schématiquement la mise en place d'une bandelette 16 à l'aide de deux implants 17, 18 d'ancrage et de fixation et deux fils 19, 20 de fixation. Les deux implants 17, 18 ont été successivement implantés dans les tissus aux endroits appropriés. S'agissant du premier implant 17, le fil de fixation 19 a ses deux extrémités libres 19a, 19b, qui sont fixées au dispositif 16. S'agissant du second implant 18, seule une extrémité libre 20a du fil de fixation 20 est fixée à la bandelette 16, délimitant avec l'implant 18 un premier brin 21 du fil de fixation 20. Entre le premier brin 21 et l'autre brin 22, qui n'est pas fixé à la bandelette 16, est formé un noeud coulissant 23. Ainsi pour mettre en tension la bandelette 16, le praticien peut exercer une traction sur l'extrémité libre 22a du second brin 22. Lors de cette traction, le noeud coulissant 23 se déplace jusqu'à ce qu'il soit suffisamment serré et bloque le second brin 22 sur le premier brin 21.

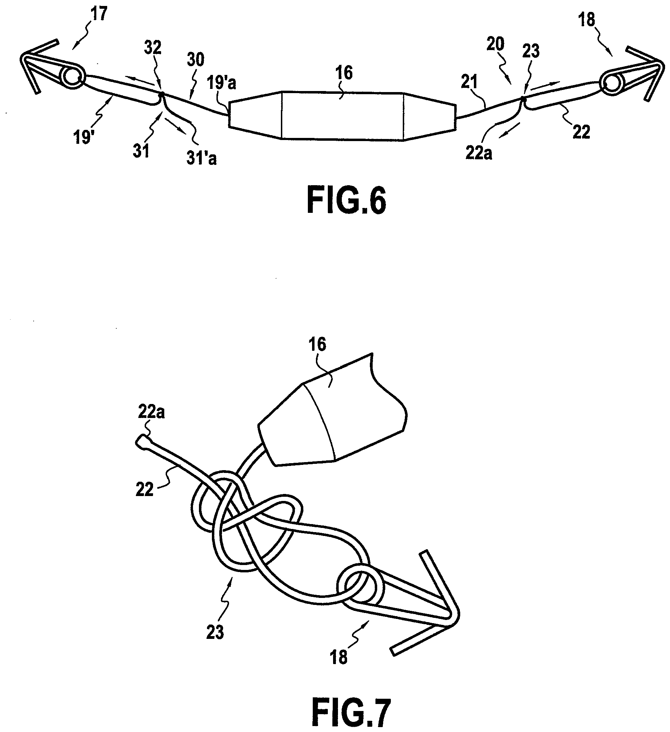

L'exemple illustré à la figure 6 diffère de celui de la figure 5 en ce que, seule une 19'a des deux extrémités libres 19'a, 19'b du fil de fixation 19' du premier implant 17 est fixée à la bandelette 16, délimitant ainsi avec l'implant 17 un premier brin de fixation 30. Entre ce premier brin 30 et l'autre brin 31, non fixée à la bandelette 16, est formé un noeud coulissant 32. Pour mettre la bandelette 16 en tension, le praticien exerce une traction sur les extrémités libres 22a, 31 a des deux brins 22 et 31, ce qui permet une plus grande possibilité de centrage de la bandelette 16 que dans l'exemple précédent.

Sur la figure 7 est illustré un mode de réalisation d'un noeud coulissant 23, tu type en huit avec boucle, qui donne toute satisfaction dans la mise en oeuvre des exemples de réalisation ci-dessus, par sa capacité de coulissement et de blocage des deux brins.

La présente invention n'est pas limitée au mode préféré de réalisation qui a été décrit ci-dessus. L'ensemble chirurgical est de préférence à usage unique mais l'ancillaire peut éventuellement être réutilisé après stérilisation. Le tube avec cavité distale qui constitue la partie utile, décrite ci-dessus, pour la mise en place de l'implant 1 peut bien sûr être assorti d'un manche de préhension et se présenter sous une forme soit rectiligne soit courbe avec possibilité de moyen d'inclinaison permettant le placement de l'implant en aveugle, son extrémité orientable lui permettant d'atteindre des espaces non directement accessibles.

## Revendications

1. Implant d'ancrage et de fixation (1) constitué d'un fil continu avec des portions rectilignes et des portions recourbées **caractérisé en ce qu'**il comporte :
a) une portion centrale recourbée (2) formant un enroulement jointif d'au moins sensiblement 1,5 spire,
b) de chaque côté et en prolongement de ladite portion centrale (2), un jeu de deux portions rectilignes (3,4), à savoir une portion intermédiaire (5,6) et une portion d'extrémité (7,8), reliées l'une à l'autre par une portion coudée (9,10) selon un angle (α) de l'ordre de 30° à 90°
et **en ce que** les portions rectilignes d'extrémité (7,8) sont orientées de manière divergente en sorte de faire entre elles un angle (β) de l'ordre de 60° à 180°, et **en ce que** le fil continu est dans un matériau à mémoire de forme en sorte que, dans une position d'introduction, les deux portions rectilignes d'extrémité (7,8) peuvent être contraintes l'une vers l'autre, de manière à former entre elles un angle de l'ordre de 10 à 30° et, dans une position d'implantation, retrouver leur position angulaire initiale de l'ordre de 60° à 180°.

2. Implant selon la revendication 1 **caractérisé en ce que** la portion centrale recourbée forme un enroulement jointif de sensiblement 1,5 spire.

3. Implant selon l'une des revendications 1 à 2, **caractérisé en ce que** la longueur des portions rectilignes d'extrémité (7,8) est inférieure à la longueur des portions rectilignes intermédiaires (5,6).

4. Implant selon l'une des revendications 1 à 3 **caractérisé en ce que** les portions rectilignes intermédiaires (5,6) font entre elles un angle (γ) de l'ordre de 10° à 30°.

5. Implant selon la revendication 4 **caractérisé en ce que** les centres des trois portions recourbées (2, 9, 10) sont dans le même plan.

6. Ensemble chirurgical (11) pour l'implantation d'un implant d'ancrage et de fixation **caractérisé en ce qu'**il comporte :
- au moins un implant (1) selon l'une des revendications 1 à 5,
- au moins un fil de fixation (13) destiné à être enfilé dans la portion centrale recourbée (2) de l'implant (1), et
- un ancillaire (12) comportant une cavité ouverte (14) à son extrémité distale, apte à servir de logement à l'implant dans une position d'introduction, dans laquelle il a sa portion centrale recourbée (2) dans le fond (15) de la cavité (14) et ses portions rectilignes d'extrémité (7,8) contraintes l'une vers l'autre en appui sur la paroi latérale de ladite cavité (14).

## Patentansprüche

1. Verankerungs- und Fixierungsimplantat (1), gebildet von einem kontinuierlichen Draht mit geraden Abschnitten und gekrümmten Abschnitten, **dadurch gekennzeichnet, dass** es aufweist:
a) einen gekrümmten zentralen Abschnitt (2), der eine verbundene Wicklung von mindestens etwa 1,5 Windungen bildet,
b) auf jeder Seite und in Verlängerung des zentralen Abschnitts (2) ein Set aus zwei geraden Abschnitten (3, 4), nämlich einem Übergangsabschnitt (5, 6) und einem Endabschnitt (7, 8), die miteinander durch einen gemäß einem Winkel (α) in der Größenordnung von 30° bis 90° angewinkelten Abschnitt (9, 10) verbunden sind,
und dass die geraden Endabschnitte (7, 8) divergierend derart ausgerichtet sind, dass sie zwischen sich einen Winkel (β) in der Größenordnung von 60° bis 180° bilden, und dass der kontinuierliche Draht aus einem Material mit Formengedächtnis derart ist, dass die zwei geraden Endabschnitte (7, 8) in einer Einführposition zueinander beanspruchbar sind, so dass sie zwischen sich einen Winkel in der Größenordnung von 10 bis 30° bilden und, in einer Implantationsposition ihre ursprüngliche Winkelposition in der Größenordnung von 60 bis 180° wiederfinden.

2. Implantat nach Anspruch 1, **dadurch gekennzeichnet, dass** der gekrümmte zentrale Abschnitt eine verbundene Wicklung von etwa 1,5 Windungen bildet.

3. Implantat nach einem der Ansprüche 1 bis 2, **dadurch gekennzeichnet, dass** die Länge der geraden Endabschnitte (7, 8) kleiner ist als die Länge der geraden Übergangsabschnitte (5, 6).

4. Implantat nach einem der Ansprüche 1 bis 3, **dadurch gekennzeichnet, dass** die geraden Obergangsabschnitte (5, 6) zwischen sich einen Winkel (γ) in der Größenordnung von 10° bis 30° bilden.

5. Implantat nach Anspruch 4, **dadurch gekennzeichnet, dass** die Zentren der drei gekrümmten Abschnitte (2, 9, 10) in derselben Ebene sind.

6. Chirurgische Einheit (11) für die Implantation eines Verankerungs- und Fixierungsimplantats, **dadurch gekennzeichnet, dass** sie aufweist:
- mindestens ein Implantat (1) nach einem der Ansprüche 1 bis 5,
- mindestens einen Fixierfaden (13), der bestimmt ist, in den gekrümmten zentralen Abschnitt (2) des Implantats (1) eingefädelt zu sein, und
- eine Hilfsvorrichtung (12), aufweisend einen an ihrem distalen Ende offenen Hohlraum (14), der imstande ist, als Aufnahme für das Implantat in einer Einführposition zu dienen, in welcher es seinen gekrümmten zentralen Abschnitt (2) im Boden (15) des Hohlraums (14) hat und seine geraden Endabschnitte (7, 8) in Abstützung auf der Seitenwand des Hohlraums (14) zueinander gespannt sind.

## Claims

1. An anchoring and fixing implant (1) constituted by a continuous thread with rectilinear portions and recurved portions, **characterised in that** it comprises :
a) a central recurved portion (2) forming a sealing winding of at least substantially 1.5 turns,
b) on either side and in extension of said central portion (2), a set of two rectilinear portions (3,4), specifically an intermediate portion (5,6) and an end portion (7, 8), connected to one another by a bent portion (9,10) at an angle (α) of the order of 30° to 90°
and **in that** the rectilinear end portions (7, 8) are oriented divergently so as to make an angle (β) of the order of 60° to 180° between them, and **in that** the continuous thread is made of shape-memory material such that, in a position of introduction, the two rectilinear end portions (7,8) can be stressed towards one another, so as to form between them an angle of the order of 10 to 30° and, in an implantation position, resume their initial angular arrangement of the order of 60° to 180°.

2. The implant according to claim 1 **characterised in that** the central recurved portion forms a sealing winding of substantially 1.5 turns.

3. The implant according to any one of claims 1 to 2, **characterised in that** the length of the rectilinear end portions (7, 8) is less than the length of the intermediate rectilinear portions (5,6).

4. The implant according to any one of claims 1 to 3 **characterised in that** the intermediate rectilinear portions (5,6) make an angle (γ) of the order of 10° to 30° between them.

5. The implant according to claim 4 **characterised in that** the centres of three recurved portions (2, 9, 10) are in the same plane.

6. A surgical assembly (11) for implantation of an anchoring and fixing implant, **characterised in that** it comprises :
- at least one implant (1) according to any one of claims 1 to 5,
- at least one fixing thread (13) intended to be threaded in the central recurved portion (2) of the implant (1), and
- an ancillary (12) comprising an open cavity (14) at its distal end, suitable for acting as housing for the implant in a position of introduction, in which it has its central recurved portion (2) in the bottom (15) of the cavity (14) and its rectilinear end portions (7, 8) stressed towards one another supported on the side wall of said cavity (14).
